**Europäisches Patentamt**

**European Patent Office**

**Office européen des brevets**

(19)

(11) Veröffentlichungsnummer: **0 019 262**
**B1**

(12) **EUROPÄISCHE PATENTSCHRIFT**

(45) Veröffentlichungstag der Patentschrift:
**15.02.84**

(21) Anmeldenummer: **80102637.8**

(22) Anmeldetag: **13.05.80**

(51) Int. Cl.³: **A 61 B 1/00**, G 02 B 23/00

(54) **Optisches System hoher Vergrösserung für Endoskope.**

(30) Priorität: **16.05.79 DE 2919677**

(43) Veröffentlichungstag der Anmeldung:
**26.11.80 Patentblatt 80/24**

(45) Bekanntmachung des Hinweises auf die Patenterteilung:
**15.02.84 Patentblatt 84/7**

(84) Benannte Vertragsstaaten:
**CH FR GB LI**

(56) Entgegenhaltungen:
**DE - A - 1 566 067**
**DE - A - 2 326 786**
**DE - A - 2 640 353**
**DE - A - 2 919 205**
**DE - B - 1 566 112**
**DE - B - 1 766 695**

(73) Patentinhaber: **Firma Carl Zeiss, Postfach 1369/1380, D-7082 Oberkochen (DE)**

(72) Erfinder: **Muchel, Franz, Dipl.-Phys., Lortzingstrasse 2, D-7923 Königsbronn (DE)**

Optisches System hoher Vergrösserung für Endoskope

Die Erfindung betrifft ein optisches System, das insbesondere für die Endoskopie geeignet ist, gemäss dem ersten Teil des Anspruchs 1. Ein derartiges System ist z. B. aus der DE-A-2 640 353 bekannt.

Endoskope bestehen ihrem allgemeinen Aufbau nach aus einem langgestreckten Rohr, das ein Bildübertragungssystem enthält und am distalen Ende mit einem Objektiv ausgerüstet ist. Proximal sind, oft abnehmbar, ein Okular, eine Kamera oder sonstige Bildaufzeichnungsgeräte angebracht.

Es ist aus der DE-B-1 566 112 bekannt, Endoskope mit Okularen zu versehen, die eine variable, auch kontinuierlich einstellbare Vergrösserung besitzen (Vario-Okulare), um eine detailliertere Beobachtung der Objektive zu ermöglichen. Diese bekannten Vario-Okulare besitzen jedoch nur einen geringen relativen Vergrösserungsfaktor, der unter 2 liegt.

Es sind aus der DE-B-1 766 695 auch Endoskope bekannt, die ein Vario-Objektiv enthalten, mit dem der Abbildungsmassstab bei feststehendem Zwischenbild durch zwei gegeneinander verschiebbare Linsen geändert wird. Auch mit diesen Vario-Objektiven wird nur eine geringe Objektvergrösserung erreicht.

Weiter ist es bekannt, ein Endoskop zu bauen, das sowohl ein Vario-Objektiv als auch ein Okular mit einer verschiebbaren Linse enthält (DE-A-2 640 353). Diese hat jedoch nur die Aufgabe, das Okular bei nahezu gleichbleibender Brennweite an die vom Vario-Objektiv beeinflusste Lage des Zwischenbildes anzupassen. Somit ist für den relativen Gesamtvergrösserungsfaktor nur die erzielbare Änderung im Abbildungsmassstab des Objektivs ausschlaggebend.

Weiter werden Endoskope benutzt, die mit einem aufgesteckten Fernrohr zur variablen Nachvergrösserung vorgesehen sind. Diese Fernrohre besitzen ein im Verhältnis zum Endoskop grosses Eigengewicht und ermöglichen somit keine bequeme Handhabung bei der Untersuchung von Patienten.

Eine signifikante Nachvergrösserung des Objekts durch Vario-Okulare allein ist bei den zur Zeit verwendeten Endoskopen prinzipiell nicht möglich, weil durch die relativ niedrigen Aperturen von herkömmlichen Endoskopobjekten die erreichbare Auflösung und die Bildhelligkeit beschränkt sind. Eine Erhöhung der Beleuchtungslichtstärke scheidet als Möglichkeit, dem letztgenannten Nachteil abzuhelfen, meist aus trivialen medizinischen Gründen aus.

Es ist die Aufgabe der vorliegenden Erfindung, ein optisches System zu schaffen, das für hochauflösende Untersuchungen in Körperöffnungen geeignet und in der Lage ist, dort Zellstrukturen sichtbar zu machen.

Gemäss dem kennzeichnenden Teil des Anspruchs 1 wird diese Aufgabe dadurch gelöst, dass

a) mindestens zwei Objektive vorgesehen sind, von denen eins eine Apertur und einen Abbildungsmassstab aufweist, damit das optische System in der Lage ist, Zellstrukturen sichtbar zu machen, und dass

b) die Objektive auswechselbar am distalen Ende des Grundkörpers anbringbar sind.

Der Vorteil der Erfindung ist darin zu sehen, dass sozusagen vor Ort, mit Hilfe eines Endoskops schnell die Untersuchungen durchgeführt werden können, für die andernfalls Gewebeproben entnommen und auf einem herkömmlichen Mikroskop untersucht werden müssten. Es ist somit auch in den Fällen ein geeignetes Untersuchungsinstrument, in denen eine Entnahme von Zellmaterial nicht möglich ist. Zum anderen erübrigt sich das Vorsehen einer komplizierten Mechanik, wie sie bei herkömmlichen Endoskopen benutzt wird, um selektiv Zellmaterial aus unzugänglichen Körperhöhlen zu entnehmen, da die Zellen bei der Untersuchung mit dem Erfindungsgegenstand nicht entnommen werden.

Die erfindungsgemässe Ausgestaltung erlaubt die wechselseitige Verwendung von Objektiven mit unterschiedlichem Abbildungsmassstab und unterschiedlicher Apertur, so dass bei erhöhtem Arbeitsabstand auch alle die Routineuntersuchungen möglich sind, für die herkömmliche Endoskope vorgesehen sind.

Das Vario-Okular erlaubt es, die Vergrösserung des Endoskops insbesondere in Verbindung mit dem hochaperturigen Objektiv in einem weiten Bereich den Erfordernissen der einzelnen Untersuchungsstadien anzupassen, da ein Objektivwechsel während der Beobachtung eines Objektes bei einer endoskopischen Zelluntersuchung kaum möglich ist. Das Vario-Okular sollte daher mindestens einen relativen Vergrösserungsfaktor von 2 besitzen.

Vorteilhaft ist auch die auswechselbare Befestigung des Okulars am Grundkörper, der dann leicht gegen einen jeweils nach Bedarf kürzeren oder längeren Grundkörper austauschbar ist. Der Grundköper kann starr oder flexibel ausgeführt sein und enthält ein optisches Bildübertragungssystem bekannter Bauart.

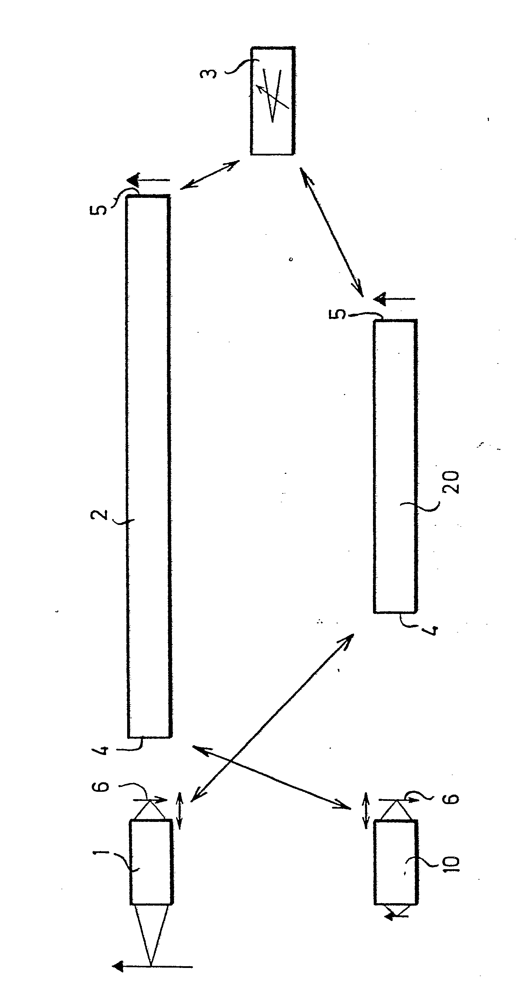

Mit Hilfe der beigefügten Zeichnung wird der Erfindungsgedanke näher erläutert.

Diese Zeichnung zeigt die Prinzipskizze eines Endoskops nach der vorliegenden Erfindung.

Mit 1 ist ein herkömmliches Endoskopobjektiv bezeichnet, das bei einer Apertur von 0,02 einen Abbildungsmassstab von − 1/3 besitzt. Dieses Objektiv kann am distalen Ende 4 eines langgestreckten Grundkörpers 2 auswechselbar befestigt werden. Der Grundkörper 2 besitzt ein Bildübertragungssystem, mit dem das vom Objektiv erzeugte Zwischenbild 6 zum proximalen Ende 5 des Grundkörpers 2 übertragen wird.

Ein Vario-Okular 3, dessen Vergrösserung kontinuierlich zwischen 9× und 45× variierbar ist,

kann am proximalen Ende 5 des Grundkörpers 2 ebenfalls auswechselbar befestigt werden.

In dieser Kombination ist bis auf das auswechselbare Objektiv ein herkömmliches Endoskop beschrieben, das für die bisher angewandten Untersuchungsmethoden geeignet ist. Der am oberen Ende der erzielbaren Gesamtvergrösserung gelegene Bereich um 15fach ist besonders für HNO-Untersuchungen von grossem Interesse.

Statt des Objektivs 1 kann auch das Objektiv 10 mit einer Apertur von 0,6 und einem Abbildungsmasstab von −25 am Grundkörper 2 befestigt werden. In dieser Kombination kann das Endoskop für die Untersuchung von Zellen verwendet werden, da der Bereich für die Gesamtvergrösserung von 250× bis 600×, in dem solche Untersuchungen normalerweise durchgeführt werden, mit Hilfe des Vario-Okulars 3 leicht überstrichen wird. Zweckmässigerweise wird in diesem Fall nicht der gesamte Bereich der Okularvergrösserung ausgenutzt, sondern nur etwa der Bereich zwischen 9× und 24×, um im Rahmen der förderlichen Vergrösserung zu bleiben.

Wie die Bezugspfeile andeuten, können die Objektive 1 bzw. 10 und das Vario-Okular 3 auch an einem Grundkörper 20 angeschlossen werden, der eine geringere Länge als der Grundkörper 2 besitzt.

Natürlich ist es auch möglich, weitere Objektive vorzusehen, deren Abbildungsmassstab so gewählt ist, dass auch die von den Objektiven 1 und 10 nicht überstrichenen Bereiche der Gesamtvergrösserung zugänglich werden.

Lösungen für die mechanische Auslegung der einzelnen Komponenten einschliesslich eines Beleuchtungssystems und eines Bildübertragungssystems zum Beispiel mit Lichtleitern sind dem Stand der Technik entnehmbar. Die spezielle Ausgestaltung der verwendeten Objektive bzw. des Vario-Okulars sind nicht Gegenstand dieser Erfindung.

## Patentansprüche

1. Optisches System für Untersuchungen in Hohlräumen, insbesondere für Endoskope, die für Untersuchungen in Körperöffnungen geeignet sind, mit einem am proximalen Ende (5) eines Grundkörpers (2, 20) angebrachten Okular (3) variabler Vergrösserung (Vario-Okular), dadurch gekennzeichnet, dass

a) mindestens zwei Objektive (1, 10) vorgesehen sind, von denen eins eine Apertur und einen Abbildungsmassstab aufweist, damit das optische System in der Lage ist, Zellstrukturen sichtbar zu machen, und dass

b) die Objektive auswechselbar am distalen Ende (4) des Grundkörpers (2, 20) anbringbar sind.

2. Optisches System nach Anspruch 1, dadurch gekennzeichnet, dass der relative Vergrösserungsfaktor des Vario-Okulars (3) grösser als 2 ist.

3. Optisches System nach Anspruch 1 und 2, dadurch gekennzeichnet, dass mindestens ein Objektiv (1) einen grösseren Abbildungsmassstab als 10 und eine höhere Apertur als 0,2 besitzt.

4. Optisches System nach Anspruch 2 bis 3, dadurch gekennzeichnet, dass mindestens zwei Grundkörper (2, 20) mit unterschiedlichen Längen vorgesehen sind, an denen die Objektive (1, 10) und das Vario-Okular (3) auswechselbar befestigbar sind.

## Claims

1. Optical system for examinations in cavities, and particularly for endoscopes, it being suitable for examinations in body cavities, with an ocular (3) of variable magnification (vario-ocular) mounted to the proximal part (5) of a base body (2, 20), characterized by the fact that

a) at least two objectives (1, 10) are provided one of which having an aperture and linear magnification that the optical system is capable of making visible cell structures, and that

b) the objectives are applicable replaceably to the distal end (4) of the base body (2, 20).

2. Optical system according to claim 1, characterized by the fact that the variable magnification factor of the vario-ocular (3) is greater than 2.

3. Optical system according to claims 1 and 2, characterized by the fact that at least one objective (1) has a linear magnification greater than 10 and an aperture higher than 0,2.

4. An optical system according to claim 2 to 3 characterized by the fact that at least two base bodies (2, 20) of different length are provided to which the objectives (1, 10) and the vario-ocular (3) are interchangeably fastened.

## Revendications

1. Système optique pour examens dans des cavités creuses, en particulier pour endoscopes convenant à des examens dans les cavités corporelles avec un oculaire (3) à grossissement variable (oculaire Vario) installé à l'extrémité image (5) d'un tube de base (2, 20), caractérisé en ce que

(a) il est prévu au moins deux objectifs (1, 10), dont l'un a une ouverture et une échelle de reproduction telles que le système optique soit à même de rendre visibles des structures cellulaires et

(b) que les objectifs puissent être installés d'une manière interchangeable à l'extrémité objet (4) du tube de base (2, 20).

2. Système optique selon la revendication 1, caractérisé en ce que le facteur de grossissement relatif de l'oculaire Vario (3) est supérieur à 2.

3. Système optique selon l'une quelconque des revendications 1 ou 2, caractérisé en ce qu'au moins un objectif (1) possède une échelle de reproduction supérieure à 10 et une ouverture supérieure à 0,2.

4. Système optique selon l'une quelconque des revendications 2 ou 3, caractérisé en ce qu'il est prévu au moins deux tubes de base (2, 20) de longueurs différentes, auxquelles on peut fixer d'une manière interchangeable les objectis (1, 10) et l'oculaire Vario (3).